# EUROPEAN PATENT SPECIFICATION

(11) **EP 4 534 049 B1**
(45) Date of publication and mention of the grant of the patent: **25.11.2026**
(21) Application number: 24204282.8
(22) Date of filing: 02.10.2024
(51) Int. Cl.: A61F 2/38

(54) **ORTHOPAEDIC SPACER MOULD**
ORTHOPÄDISCHE DISTANZFORM
MOULE D'ESPACEMENT ORTHOPÉDIQUE

(30) Priority: 05.10.2023 GB 202315285
(43) Date of publication of application: 09.04.2025
(73) Proprietor: Vyrides, Yiannis, Nicosia 1075 (CY)
(72) Inventor: Vyrides, Yiannis, Nicosia 1075 (CY)
(74) Representative: Cameron Intellectual Property Ltd

(56) References cited:
- EP-B1- 3 143 963
- CN-A- 107 095 729
- CN-A- 111 839 837
- US-A1- 2010 102 484
- US-A1- 2013 295 213
- US-A1- 2021 290 395

## Description

### Technical Field

The present disclosure relates to orthopaedic spacer moulding, and particularly, but not exclusively, orthopaedic spacer moulding for a femoral spacer.

### Background

Joint replacement surgery, for example knee replacement surgery is increasingly common. While many knee replacements are successful, a small percentage of knee replacements become infected, sometimes resulting in the need to remove the infected prosthetic joint (and any other foreign material) followed by a prolonged antibiotic course. Once the infection has been eradicated then re-implantation of a new prosthetic joint can be undertaken.

In such situations, the new prosthetic joint is not implanted until the infection itself is no longer present in the body which can require weeks or months of treatment, for example using antibiotics. So, between the operation to remove the infected prosthetic joint and the operation in which the new prosthetic joint is implanted, an orthopaedic spacer is implanted in the place vacated by the infected prosthetic joint.

The spacer fills the area of the prosthetic joint ensuring that the area remains at the same size to receive the future replacement. In the case of a knee replacement, the spacer can also allow the patient some use of their leg while the infection is treated.

Orthopaedic spacers can be bought in a limited number of sizes. Alternatively, spacers can be manually formed or modified using surgical cement, but such processes can lengthen time spent in surgery and produce spacers of variable quality.

European Patent number EP 3143963 B1 discloses a method and device for producing temporary knee joint spacers with stems of various sizes, as in the preamble of claim 1.

### Summary

There is provided a mould for casting a spacer for a joint replacement, the mould comprising a mould body comprising a cavity wall, the mould body closable to define a cavity with the cavity wall, wherein the mould is adjustable between a plurality of configurations, wherein in each of the configurations, the cavity is restricted to a respective different size. The mould allows a user to select a size for the spacer and use the mould to quickly and reliably produce an appropriate spacer. The mould may be referred to as an orthopaedic spacer mould.

Optionally, when the mould is closed, the cavity has an opening enabling surgical cement to be inserted into the cavity, for example via a surgical cement applicator nozzle. In this specification, the term cement refers to surgical cement.

The plurality of configurations are discrete configurations. There may be between 3 and 10 discrete configurations, for example 6. In this application, the term discrete has its usual meaning and is used in contrast to a continuous adjustment, such as a mechanism in which the size may be any value between a minimum and maximum. Discrete configurations allow easier selection of an appropriate size.

Optionally, the mould body may be in two halves.

Optionally, in each of the configurations, the cavity is restricted to a respective different volume and/or length. In this specification, size may refer to volume and/or length.

Optionally, the cavity wall is shaped such that the cavity comprises a shaft portion, optionally, a metaphysis portion and optionally, an epiphysis portion, for example, a distal epiphysis portion.

Optionally, in each of the plurality of configurations, the shaft portion of the cavity is restricted to a different length. The length of the shaft portion is a dimension in the direction of a longitudinal axis of the shaft. The epiphysis portion may remain the same size/length/volume.

The mould further comprises an end cap and in each of the plurality of configurations, the end cap is positioned at a different respective position relative to the mould body.

The end cap has an aperture therethrough. In use, a cement applicator nozzle may be inserted through the aperture. The aperture may be in a centre of the end cap and/or may align with a central longitudinal axis of the shaft portion of the cavity when in one or more or all of the configurations.

Optionally, the mould body comprises a plurality of notches, spaced from each other along the cavity wall, each of the notches being configured to receive the end cap, wherein the end cap is positionable in each one of the notches, to thereby form each of the plurality of configurations of the mould. The notches may comprise one or more grooves. For example, when the mould body is formed of two halves, the notches may each comprise a pair of grooves in each half of the mould body, optionally each groove matching a portion of an outer edge or circumference of the end cap. For example, if the end cap is circular, the grooves may each form half of a circular notch in the cavity wall. Each groove can then receive half of an outer edge or circumference of the end cap.

When the mould body is in two halves, in use, the end cap may be positioned in a first groove in a first half of the mould body, then the second half of the mould body is positioned against the first half of the mould body and the end cap so that the end cap is also received in the second groove in the second half of the mould body, to thereby hold the end cap in place between the halves of the mould body. In this application, halves of the mould body are described, but it should be recognised that the halves need not be the same size as each other.

Optionally, the mould is a femoral spacer mould, for example, a distal femoral spacer mould.

Optionally, the mould body further defines a plurality of riser sub-cavities, the riser sub-cavities being elongate and optionally extending diagonally from a portion of the cavity wall shaped to form the shaft portion of the cavity in a direction away from a portion of the cavity wall shaped to form the epiphysis portion of the cavity.

Optionally, the mould body comprises a measuring scale disposed on the mould body alongside the cavity wall. The measuring scale may be disposed alongside the possible positions of the end cap to enable easy selection of the required size of spacer.

Optionally the notches are spaced along a portion of the cavity wall shaped to form the shaft portion of the cavity. The notches may be spaced longitudinally along a portion of the cavity wall shaped to form the shaft portion of the cavity. In this way, the end cap may be placed in one of the notches to form a spacer with a selected shaft length.

Optionally, the cavity wall extends to an outward facing surface of the mould body. Optionally, a portion of the cavity wall shaped to form the shaft portion of the cavity extends to an outward facing surface of the mould body. In this way an opening is provided to the cavity enabling insertion of cement.

The mould body halves may each have a contact surface configured to contact the other of the mould body halves when the mould is closed. The contact surfaces may have reciprocal profiles to each other. The contact surfaces may be non-planar. This ensures effective alignment of the mould body halves when the mould is closed. The contact surfaces may be shaped so that at each position along the longitudinal axis of the shaft portion of the cavity, between 30 and 70% of the cavity wall is provided by each of the mould body halves. This reduces the likelihood of the resultant spacer becoming stuck in one half of the mould body if one half of the mould body were to produce all or nearly all of the spacer.

The contact surface of the first mould body half may comprise a protrusion such as a dowel and the contact surface of the second mould half may comprise a corresponding indent. This enables the mould body to be properly aligned when in use. Optionally, a hole may be provided through one the first mould body half and an aligned hole may be provided in the second mould body half. The holes may be sized to accept screws, for example M8 screws, so that the mould body can be secured together by screws whilst the cement sets. The holes may be configured to accept M8x 1.25 screws. The holes may be configured to accept thumb screws. The screws may be provided as part of the mould. Optionally, two sets of such holes are provided in different positions on the mould.

There is provided a method of forming a spacer for a joint replacement, using a mould as described above. The method comprises: adjusting the mould to restrict the cavity to a selected size; closing the mould body to define the cavity; inserting cement into the mould; waiting until the cement has set; and opening the mould to release the formed spacer.

Optionally, adjusting the mould to restrict the cavity to a selected size comprises restricting the length of a shaft portion of the mould. Optionally, adjusting the mould to restrict the cavity to a selected size comprises positioning an end cap in a position in the mould body corresponding to the selected size. Optionally, adjusting the mould to restrict the cavity to a selected size comprises positioning the end cap in a selected one of a plurality of notches on the cavity wall.

Optionally, closing the mould body comprises securing the mould body in a closed position, optionally using screws, such as thumbs screws.

Optionally, inserting cement into the mould comprises inserting cement into the mould through an open end of the cavity on an outward facing surface of the mould body. Optionally, inserting cement into the mould comprises inserting cement into the mould through an aperture in the end cap. For example, this may be performed using an applicator nozzle for surgical cement.

Optionally, the method further comprises removing elongate cement risers from the spacer.

Optionally, the cement comprises antibiotics.

The methods and moulds described above may be combined in any possible combination. The optional features described above are equally applicable to all of the described methods and moulds and are not limited to the particular method/mould with which they are described here. The essential features of any of the methods/moulds described may be optional features of any other methods/moulds described.

Further features and advantages of the aspects of the present disclosure will become apparent from the claims and the following description.

### Brief Description of Drawings

Embodiments of the present disclosure will now be described by way of example only, with reference to the following diagrams, in which:-
Figure 1 is a perspective view of an orthopaedic spacer mould;
Figure 2 is a perspective view of a portion of a mould body of the orthopaedic spacer mould of Figure. 1;
Figure 3 is a perspective view of an end cap of the orthopaedic spacer mould of Figure 1;
Figure 4 is a perspective view of a portion of a mould body of the orthopaedic spacer mould of Figure. 1.

### Detailed Description

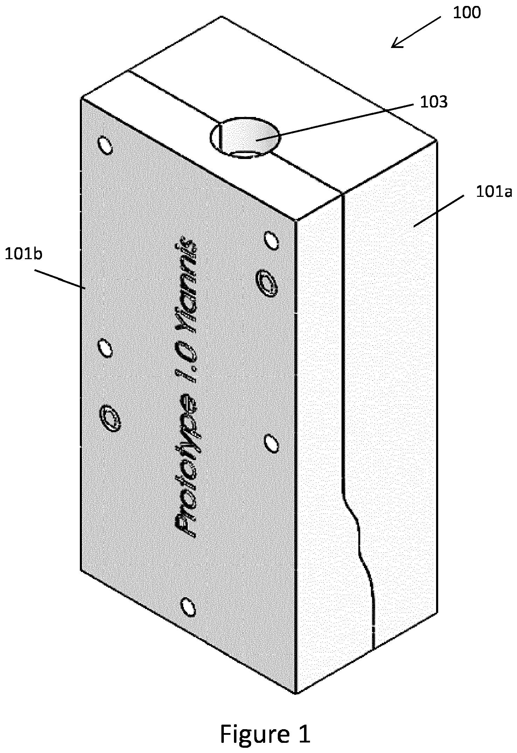

With reference to Figs. 1 to 4 an orthopaedic spacer mould will now be described.

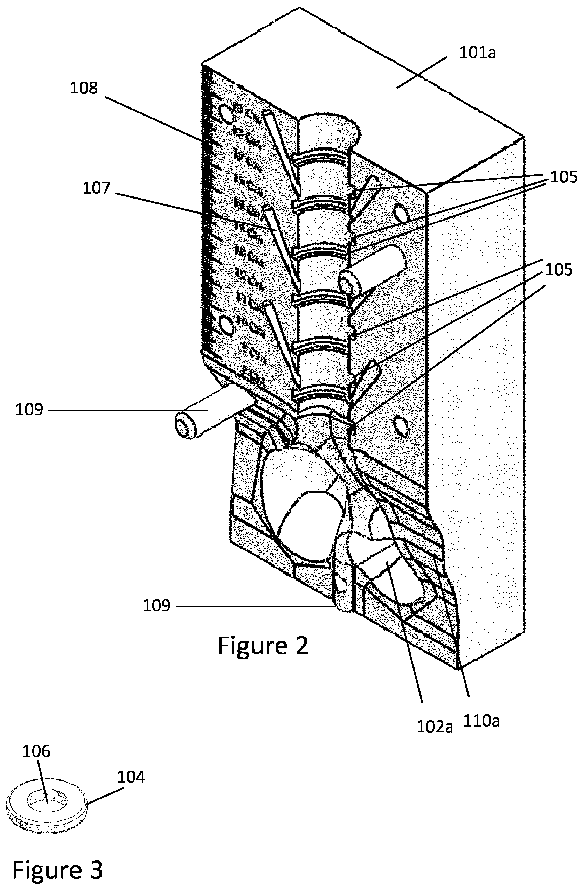

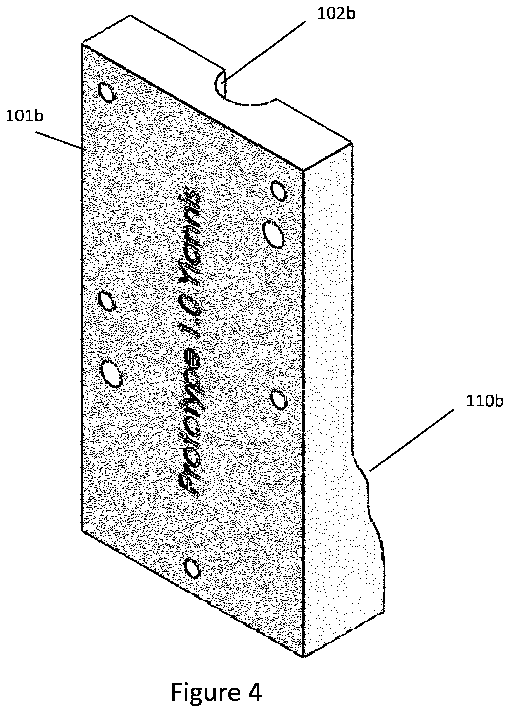

Figure 1 shows a mould 100 for casting a spacer for a joint replacement. The mould comprises a mould body formed of two halves 101a, 101b comprising a cavity wall 102a, 102b. The mould body 101a, 101b is closable, as shown in Figure 1, to define a cavity 103 with the cavity wall 102a, 102b. A first half of the mould body 101a is shown in Figure 2 and the second half 101b is shown in Figure 4.

The mould 100 is an orthopaedic femoral spacer mould and the cavity wall 102a, 102b is shaped such that the cavity 103 comprises a shaft portion and an epiphysis portion.

The mould 100 is adjustable between a plurality of configurations and in each of the configurations, the cavity 103 is restricted to a respective different size. In order to provide the plurality of configurations, the mould body 101a, 101b, has a plurality of notches 105, longitudinally spaced from each other along the cavity wall 102a, 102b. Each of the notches are configured to receive the end cap 104, shown in Figure 3. The end cap 104 is positionable in each one of the notches 105, to thereby form each of the plurality of configurations of the mould 100.

The notches 105 are each formed by a groove in the first half of the mould 101a and a corresponding groove in the second half of the mould 102b. Each notch 105 is sized to receive an outer edge of the end cap 104. In this embodiment, the end cap 104 is circular, so the grooves each form half of a circular notch. Each groove can then receive half of an outer edge of the end cap 104.

In use, the end cap 104 is positioned in the selected groove of one of the halves of the mould body 101a, 101b, then the other half of the mould body 101a, 101b is positioned against the first half of the mould body and the end cap, so that the end cap 104 is also received in both halves of the selected notch to thereby hold the end cap 104 in place between the halves of the mould body.

Due to the spacing of the notches, the plurality of configurations are discrete. In this example, 6 discrete configurations are provided by the 6 spaced notches.

It can be seen that in each of the configurations with the end cap placed in a respective different notch, the cavity 103 is restricted to a respective different length. As the notches are located on a shaft portion of the cavity wall 102a, 102b, the shaft portion of the cavity 103 is restricted to a different length in each configuration. The epiphysis portion remains the same size, length and volume in each configuration.

The portion of cavity wall 102a, 102b shaped to form the shaft portion of the cavity extends to an outward facing surface of the mould body to thereby form an opening when the mould body is closed. The opening enables surgical cement to be inserted into the cavity 103, via a surgical cement applicator nozzle. Further, the end cap 104 has an aperture 106 therethrough. In use, a cement applicator nozzle can be inserted through the opening and through the aperture 106 to allow precise application of the cement into the restricted cavity. The aperture 104 is in the centre of the end cap 104 and when the end cap 104 is in any one of the notches 105, the aperture 106 aligns with a centre axis of the shaft portion of the cavity 103.

The mould body further defines a plurality of riser sub-cavities 107. The riser sub-cavities are elongate and extend diagonally from the portion of the cavity wall shaped to form the shaft portion of the cavity in a direction away from a portion of the cavity wall shaped to form the epiphysis portion. Such riser sub-cavities allow air to escape when cement is applied to reduce likelihood of air bubbles in the resultant spacer and ensure the restricted cavity is filled with cement.

The mould body further comprises a measuring scale 108 disposed on the mould body alongside the shaft portion of the cavity wall 102a of the first half of the mould body. The measuring scale is disposed alongside the notches to enable easy selection of the required length of spacer.

The mould body halves each have a contact surface 110a, 110b configured to contact the other of the mould body halves when the mould is closed. The contact surfaces 110a, 110b have reciprocal profiles. The contact surfaces are non-planar. This ensures effective alignment of the mould body halves wen the mould is closed. The contact surfaces may be shaped so that at each height relative to the longitudinal axis of the shaft, between 30 and 70% of the cavity wall is provided by each of the mould body halves. This reduces the likelihood of the resultant spacer becoming stuck in one half of the mould body.

The contact surface 110a of the first mould body half comprises protrusions 109 and the contact surface 110b of the second mould half comprises corresponding indents. This enables the mould body to be properly aligned when in use.

A method of forming a spacer for a joint replacement using a mould as described above will now be described.

Firstly, the end cap 104 is placed in the selected groove in the first half of mould body 101a to restrict the length of a shaft portion of the mould to the selected size. The second half of the mould body 101b is then positioned against the first half 101a and the end cap 104 to thereby close the mould body and position the end cap in a selected one of a plurality of notches 105 on the cavity wall 102a, 102b. The closed mould thereby defines the cavity 103 and restricts the cavity via the position of end cap 104.

Cement is then inserted in the mould through the opening of cavity 103 on an outward facing surface of the mould body and through aperture 106 in the end cap 104. This may be performed using an applicator nozzle for surgical cement. The cement comprises antibiotics.

Once the cement has set, the mould 100 is opened to release the formed spacer and any elongate cement risers which may have been formed in sub-cavities 107 are removed..

Although particular embodiments of the disclosure have been disclosed herein in detail, this has been done by way of example and for the purposes of illustration only. The aforementioned embodiments are not intended to be limiting with respect to the scope of the appended claims.

It is contemplated by the inventors that various substitutions, alterations, and modifications may be made to the embodiment(s) without departing from the scope of the invention as defined by the claims.

## Claims

1. A mould (100) for casting a spacer for a joint replacement, the mould comprising a mould body (101a, 101b) comprising a cavity wall (102a, 102b), the mould body closable to define a cavity (103) with the cavity wall,
wherein the mould (100) is adjustable between a plurality of discrete configurations,
wherein in each of the configurations, the cavity (103) is restricted to a respective different size,
wherein the mould further comprises an end cap (104) and in each of the plurality of configurations, the end cap (104) is positioned at a different respective position relative to the mould body (101a, 101b), and
**characterised in that** the end cap (104) has an aperture therethrough.

2. The mould (100) of claim 1, wherein the cavity wall(102a, 102b) is shaped such that the cavity (103) comprises a shaft portion, a metaphysis portion and an epiphysis portion.

3. The mould (100) of claim 2, wherein in each of the plurality of configurations, the shaft portion of the cavity (103) is restricted to a different length.

4. The mould (100) of any preceding claim, wherein the mould body (101a, 101b) comprises a plurality of notches (105), spaced from each other along the cavity wall (102a, 102b), each of the notches being configured to receive the end cap (104), wherein the end cap (104) is positionable in each one of the notches (105), to thereby form each of the plurality of configurations of the mould.

5. The mould (100) of claim 4, when dependent on claims 2 and 3, wherein the notches (105) are spaced along a portion of the cavity wall (102a, 102b) shaped to form the shaft portion of the cavity (103).

6. The mould (100) of any preceding claim, wherein the mould is a distal femoral spacer mould.

7. The mould (100) of claim 2, or any of claims 3 to 6 when dependent on claim 2, wherein the mould body (101a, 101b) further defines a plurality of riser sub-cavities (107), the riser sub-cavities (107) being elongate and extending diagonally from a portion of the cavity wall (102a, 102b) shaped to form the shaft portion of the cavity (103) in a direction away from a portion of the cavity wall (102a, 102b) shaped to form the epiphysis portion.

8. The mould (100) of any preceding claim, wherein the mould body (101a, 101b) comprises a measuring scale (108) disposed on the mould body alongside the cavity wall (102a, 102b).

9. The mould (100) of any preceding claim, wherein the cavity wall (102a, 102b) extends to an outward facing surface of the mould body (101a, 101b).

10. A method of forming a spacer for a joint replacement using the mould of any of claims 1 to 9, the method comprising:
adjusting the mould to restrict the cavity to a selected size;
closing the mould body to define the cavity;
inserting cement into the mould;
waiting until the cement has set; and
opening the mould to release the formed spacer.

11. A method according to claim 10, wherein adjusting the mould to restrict the cavity to a selected size comprises restricting the length of a shaft portion of the mould.

12. A method according to claim 10 or 11, wherein adjusting the mould to restrict the cavity to a selected size comprises positioning an end cap in a position in the mould body corresponding to the selected size.

13. A method according to claim 12, wherein inserting cement into the mould comprises inserting cement into the mould through an aperture in the end cap.

14. A method according to any one of claims 12 or 13, wherein adjusting the mould to restrict the cavity to a selected size comprises positioning the end cap in a selected one of a plurality of notches on the cavity wall.

15. A method according to any of claims 10 to 14,
wherein the method further comprises removing elongate cement risers from the spacer, and/or,
wherein inserting cement into the mould comprises inserting cement into the mould through an open end of the cavity on an outward facing surface of the mould body, and/or,
wherein the cement comprises antibiotics.

## Patentansprüche

1. Gießform (100) zum Gießen eines Spacers für einen Gelenkersatz, wobei die Gießform einen Gießformkörper (101a, 101b) umfasst, der eine Hohlraumwand (102a, 102b) umfasst, wobei der Gießformkörper schließbar ist, um mit der Hohlraumwand einen Hohlraum (103) zu definieren,
wobei die Gießform (100) zwischen einer Mehrzahl diskreter Konfigurationen verstellbar ist,
wobei in jeder der Konfigurationen der Hohlraum (103) auf eine jeweils unterschiedliche Größe begrenzt ist,
wobei die Gießform ferner eine Endkappe (104) umfasst und in jeder der Mehrzahl von Konfigurationen die Endkappe (104) an einer jeweils anderen Position relativ zu dem Gießformkörper (101a, 101b) positioniert ist, und
**dadurch gekennzeichnet, dass** die Endkappe (104) eine durch sie hindurchgehende Öffnung aufweist.

2. Gießform (100) nach Anspruch 1, wobei die Hohlraumwand (102a, 102b) so geformt ist, dass der Hohlraum (103) einen Schaftabschnitt, einen Metaphysenabschnitt und einen Epiphysenabschnitt umfasst.

3. Gießform (100) nach Anspruch 2, wobei in jeder der Mehrzahl von Konfigurationen der Schaftabschnitt des Hohlraums (103) auf eine unterschiedliche Länge begrenzt ist.

4. Gießform (100) nach einem der vorhergehenden Ansprüche, wobei der Gießformkörper (101a, 101b) eine Mehrzahl von Kerben (105) umfasst, die entlang der Hohlraumwand (102a, 102b) voneinander beabstandet sind, wobei jede der Kerben dazu ausgebildet ist, die Endkappe (104) aufzunehmen, wobei die Endkappe (104) in jeder der Kerben (105) positionierbar ist, um dadurch jede der Mehrzahl von Konfigurationen der Gießform zu bilden.

5. Gießform (100) nach Anspruch 4, sofern auf die Ansprüche 2 und 3 rückbezogen, wobei die Kerben (105) entlang eines Abschnitts der Hohlraumwand (102a, 102b) beabstandet sind, der zum Bilden des Schaftabschnitts des Hohlraums (103) geformt ist.

6. Gießform (100) nach einem der vorhergehenden Ansprüche, wobei die Gießform eine Gießform für einen distalen Femur-Spacer ist.

7. Gießform (100) nach Anspruch 2 oder nach einem der Ansprüche 3 bis 6, sofern auf Anspruch 2 rückbezogen, wobei der Gießformkörper (101a, 101b) ferner eine Mehrzahl von Steiger-Teilhohlräumen (107) definiert, wobei die Steiger-Teilhohlräume (107) länglich sind und sich diagonal von einem Abschnitt der Hohlraumwand (102a, 102b), der zum Bilden des Schaftabschnitts des Hohlraums (103) geformt ist, in einer Richtung weg von einem Abschnitt der Hohlraumwand (102a, 102b) erstrecken, der zum Bilden des Epiphysenabschnitts geformt ist.

8. Gießform (100) nach einem der vorhergehenden Ansprüche, wobei der Gießformkörper (101a, 101b) eine Messskala (108) umfasst, die an dem Gießformkörper entlang der Hohlraumwand (102a, 102b) angeordnet ist.

9. Gießform (100) nach einem der vorhergehenden Ansprüche, wobei sich die Hohlraumwand (102a, 102b) bis zu einer nach außen gewandten Oberfläche des Gießformkörpers (101a, 101b) erstreckt.

10. Verfahren zum Formen eines Spacers für einen Gelenkersatz unter Verwendung der Gießform nach einem der Ansprüche 1 bis 9, wobei das Verfahren umfasst:
Verstellen der Gießform, um den Hohlraum auf eine ausgewählte Größe zu begrenzen;
Schließen des Gießformkörpers, um den Hohlraum zu definieren;
Einbringen von Zement in die Gießform;
Warten, bis der Zement ausgehärtet ist; und
Öffnen der Gießform, um den geformten Spacer freizugeben.

11. Verfahren nach Anspruch 10, wobei das Verstellen der Gießform, um den Hohlraum auf eine ausgewählte Größe zu begrenzen, ein Begrenzen der Länge eines Schaftabschnitts der Gießform umfasst.

12. Verfahren nach Anspruch 10 oder 11, wobei das Verstellen der Gießform, um den Hohlraum auf eine ausgewählte Größe zu begrenzen, ein Positionieren einer Endkappe an einer der ausgewählten Größe entsprechenden Position in dem Gießformkörper umfasst.

13. Verfahren nach Anspruch 12, wobei das Einbringen von Zement in die Gießform ein Einbringen von Zement in die Gießform durch eine Öffnung in der Endkappe umfasst.

14. Verfahren nach einem der Ansprüche 12 oder 13, wobei das Verstellen der Gießform, um den Hohlraum auf eine ausgewählte Größe zu begrenzen, ein Positionieren der Endkappe in einer ausgewählten Kerbe aus einer Mehrzahl von Kerben an der Hohlraumwand umfasst.

15. Verfahren nach einem der Ansprüche 10 bis 14,
wobei das Verfahren ferner ein Entfernen länglicher Zementsteiger von dem Spacer umfasst, und/oder,
wobei das Einbringen von Zement in die Gießform ein Einbringen von Zement in die Gießform durch ein offenes Ende des Hohlraums an einer nach außen gewandten Oberfläche des Gießformkörpers umfasst, und/oder,
wobei der Zement Antibiotika umfasst.

## Revendications

1. Moule (100) pour couler un espaceur pour un remplacement articulaire, le moule comprenant un corps de moule (101a, 101b) comprenant une paroi de cavité (102a, 102b), le corps de moule pouvant être fermé pour définir une cavité (103) avec la paroi de cavité,
dans lequel le moule (100) est réglable entre une pluralité de configurations discrètes,
dans lequel, dans chacune des configurations, la cavité (103) est restreinte à une taille différente respective,
dans lequel le moule comprend en outre un capuchon d'extrémité (104) et, dans chacune de la pluralité de configurations, le capuchon d'extrémité (104) est positionné à une position respective différente par rapport au corps de moule (101a, 101b), et
**caractérisé en ce que** le capuchon d'extrémité (104) présente une ouverture le traversant.

2. Moule (100) selon la revendication 1, dans lequel la paroi de cavité (102a, 102b) est conformée de telle sorte que la cavité (103) comprend une partie de tige, une partie métaphysaire et une partie épiphysaire.

3. Moule (100) selon la revendication 2, dans lequel, dans chacune de la pluralité de configurations, la partie de tige de la cavité (103) est restreinte à une longueur différente.

4. Moule (100) selon l'une quelconque des revendications précédentes, dans lequel le corps de moule (101a, 101b) comprend une pluralité d'encoches (105), espacées les unes des autres le long de la paroi de cavité (102a, 102b), chacune des encoches étant configurée pour recevoir le capuchon d'extrémité (104), dans lequel le capuchon d'extrémité (104) peut être positionné dans chacune des encoches (105), pour former ainsi chacune de la pluralité de configurations du moule.

5. Moule (100) selon la revendication 4, lorsqu'elle dépend des revendications 2 et 3, dans lequel les encoches (105) sont espacées le long d'une partie de la paroi de cavité (102a, 102b) conformée pour former la partie de tige de la cavité (103).

6. Moule (100) selon l'une quelconque des revendications précédentes, dans lequel le moule est un moule d'espaceur fémoral distal.

7. Moule (100) selon la revendication 2, ou selon l'une quelconque des revendications 3 à 6 lorsqu'elle dépend de la revendication 2, dans lequel le corps de moule (101a, 101b) définit en outre une pluralité de sous-cavités de masselotte (107), les sous-cavités de masselotte (107) étant allongées et s'étendant en diagonale à partir d'une partie de la paroi de cavité (102a, 102b) conformée pour former la partie de tige de la cavité (103) dans une direction s'éloignant d'une partie de la paroi de cavité (102a, 102b) conformée pour former la partie épiphysaire.

8. Moule (100) selon l'une quelconque des revendications précédentes, dans lequel le corps de moule (101a, 101b) comprend une échelle de mesure (108) disposée sur le corps de moule le long de la paroi de cavité (102a, 102b).

9. Moule (100) selon l'une quelconque des revendications précédentes, dans lequel la paroi de cavité (102a, 102b) s'étend jusqu'à une surface orientée vers l'extérieur du corps de moule (101a, 101b).

10. Procédé de formation d'un espaceur pour un remplacement articulaire au moyen du moule selon l'une quelconque des revendications 1 à 9, le procédé comprenant :
le réglage du moule pour restreindre la cavité à une taille sélectionnée ;
la fermeture du corps de moule pour définir la cavité ;
l'introduction de ciment dans le moule ;
l'attente jusqu'à ce que le ciment ait fait prise ; et
l'ouverture du moule pour libérer l'espaceur formé.

11. Procédé selon la revendication 10, dans lequel le réglage du moule pour restreindre la cavité à une taille sélectionnée comprend la restriction de la longueur d'une partie de tige du moule.

12. Procédé selon la revendication 10 ou 11, dans lequel le réglage du moule pour restreindre la cavité à une taille sélectionnée comprend le positionnement d'un capuchon d'extrémité dans une position dans le corps de moule correspondant à la taille sélectionnée.

13. Procédé selon la revendication 12, dans lequel l'introduction de ciment dans le moule comprend l'introduction de ciment dans le moule à travers une ouverture dans le capuchon d'extrémité.

14. Procédé selon l'une quelconque des revendications 12 ou 13, dans lequel le réglage du moule pour restreindre la cavité à une taille sélectionnée comprend le positionnement du capuchon d'extrémité dans une encoche sélectionnée parmi une pluralité d'encoches sur la paroi de cavité.

15. Procédé selon l'une quelconque des revendications 10 à 14,
dans lequel le procédé comprend en outre le retrait de masselottes de ciment allongées de l'espaceur, et/ou,
dans lequel l'introduction de ciment dans le moule comprend l'introduction de ciment dans le moule à travers une extrémité ouverte de la cavité sur une surface orientée vers l'extérieur du corps de moule, et/ou,
dans lequel le ciment comprend des antibiotiques.
